# EUROPÄISCHE PATENTSCHRIFT

(11) **EP 1 099 685 B1**
(45) Veröffentlichungstag und Bekanntmachung des Hinweises auf die Patenterteilung: **04.02.2004**
(21) Anmeldenummer: 00123322.0
(22) Anmeldetag: 27.10.2000
(51) Int. Cl.: C07C 205/12, C07C 201/08

(54) **Verfahren zur adiabatischen Herstellung von 3,4-Dichlornitrobenzol**
Process for the adiabatic preparation of 3,4-dichloronitrobenzene
Procédé pour la préparation adiabatique de 3,4-dichloronitrobenzène

(30) Priorität: 09.11.1999 DE 19953722
(43) Veröffentlichungstag der Anmeldung: 16.05.2001
(73) Patentinhaber: Bayer Chemicals AG, 51368 Leverkusen (DE)
(72) Erfinder: Zirngiebl, Eberhard, Dr., 51061 Köln (DE); König, Bernd-Michael, Dr., 51467 Bergisch Gladbach (DE); Weber, Hans-Martin, Dr., 51373 Leverkusen (DE); Linn, Thomas, Dr., 41515 Grevenbroich (DE); Raatz, Hans-Joachim, 51381 Leverkusen (DE)

(56) Entgegenhaltungen:
- EP-A- 0 675 104
- US-A- 2 256 999
- US-A- 4 021 498
- US-A- 4 453 027

## Beschreibung

Die vorliegende Erfindung betrifft ein Verfahren zur hochselektiven, abfallsäurefreien Herstellung von 3,4-Dichlornitrobenzol unter Ausnutzung der Reaktionswärme.

3,4-Dichlornitrobenzol ist ein wichtiges Zwischenprodukt zur Herstellung von Pflanzenschutzmitteln und Farbstoffen.

3,4-Dichlornitrobenzol wird technisch durch isotherme Nitrierung von 1,2-Dichlorbenzol bei Temperaturen zwischen 40 und 70°C hergestellt (Ullmann's Encyclopedia of Industrial Chemistry, 5^{th} Edition, Vol. A17, p. 430, 1991). Dabei fallen große Mengen verunreinigter Abfallsäure an, die kostenintensiv entsorgt oder aufgearbeitet werden müssen. Nachteilig bei diesen Verfahren ist, dass eine erhebliche Reaktionswärme sicher abgeführt werden muss, was zu hohen Anforderungen an die zu realisierende Sicherheitstechnik führt.

Um den Anfall von Abfallsäure zu vermeiden, sind Verfahren anzustreben, die eine integrierte Schwefelsäureaufkonzentrierung unter Ausnutzung der Reaktionswärme beinhalten. Dies bedingt eine Kreislaufsäure, in der sich Nebenprodukte nicht anreichern dürfen.

Die oben genannten Nachteile lassen sich dadurch umgehen, dass das Nitrierverfahren adiabatisch durchgeführt wird. Die adiabatische Mononitrierung ist bereits für verschiedene aromatische Verbindungen beschrieben.

Adiabatische Nitrierverfahren zeichnen sich dadurch aus, daß die entstehende Reaktionswärme nicht durch eine aufwendige Kühlung ständig zuverlässig abgeführt werden muss, sondern bestimmungsgemäß im Reaktionssystem verbleibt. Die oben genannten energetischen und sicherheitstechnischen Nachteile bei der isothermen Nitrierung von 1,2-Dichlorbenzol entfallen also.

Die Reaktionswärme kann zur Erhitzung der Kreislauf-Schwefelsäure und zur Erleichterung der Aufkonzentrierung der Säure benutzt werden.

Ein weiterer Vorteil der adiabatischen Reaktionsführung besteht darin, dass die Reaktionsgeschwindigkeit hoch ist: die Reaktionen sind nach deutlich unter 10 Minuten beendet.

Überdies ist es möglich, kostengünstige Schwachsäure (HNO₃ ca. 60-65 %ig) einzusetzen.

In EP 668 263 A1 ist ein Verfahren zur adiabatischen Herstellung von Mononitrotoluolen beschrieben. Es wird ein Gemisch aus Toluol, Salpetersäure, Schwefelsäure und Wasser unter adiabatischen Reaktionsbedingungen umgesetzt, wobei die Reaktionskomponenten intensiv vermischt werden und die Temperatur während der Vermischung zwischen 20 und 110 °C liegt.

Auch die adiabatische Mononitrierung von 1,2-Dichlorbenzol ist bereits bekannt.

Ein adiabatisches Nitrierverfahren zur Herstellung von Mononitrohalogenbenzolen wird in US 4 453 027 beansprucht. Die dort beschriebenen Bedingungen sind für eine sinnvolle technische Herstellung von 3,4-Dichlomitrobenzol jedoch ungeeignet. Die verwendete Nitriersäure enthält 11,2 Gew.-% HNO₃, 68,5 Gew.-% H₂SO₄ und 20,3 Gew.-% H₂O. Der hohe Gehalt an HNO₃ bedingt bei adiabatischer Reaktionsführung eine Temperaturerhöhung während der Reaktion von ca. 100 °C, sodass die Temperatur am Ende der Reaktion 100 °C deutlich übersteigt, selbst wenn die Vermischung der Reaktionskomponenten beispielsweise bei einer Temperatur von nur 45 °C erfolgt. Dadurch wird bei der Herstellung von 3,4-Dichlomitrobenzol aus 1,2-Dichlorbenzol vermehrt das unerwünschte Nebenprodukt 2,3-Dichlornitrobenzol gebildet.

EP 675 104 A1 offenbart ein Verfahren zur adiabatischen Mononitrierung von Mononitrohalogenbenzolen, wobei die Nitrierung von 1,2-Dichlorbenzol explizit genannt ist. Die adiabatische Nitrierung wird so durchgeführt, dass die Reaktionskomponenten intensiv vermischt werden, die Temperatur während der Vermischung zwischen 60 und 160 °C liegt und die Temperatur am Ende der Reaktion 180 °C nicht übersteigt. Nach diesem Verfahren läßt sich 1,2-Dichlorbenzol zu 3,4-Dichlomitrobenzol umsetzen. Es werden jedoch über 16 Gew.-% (bezogen auf die Gesamtmenge an Dichlornitrobenzolen) des unerwüschten Nebenprodukts 2,3-Dichlornitrobenzol gebildet, das in vielen Fällen kostenintensiv entsorgt oder nicht kostendeckend verkauft werden muss.

Es bestand daher Bedarf an einem Verfahren, das die sicherheitstechnischen und ökonomischen Vorteile bezüglich Einsatzstoffen und Energieausnutzung eines adiabatischen Verfahrens mit einer hohen Selektivität bezüglich der Bildung von 3,4-Dichlomitrobenzol verbindet.

Es wurden nun Reaktionsbedingungen gefunden, bei denen sich die widersprechenden Forderungen einer niedrigen Nebenproduktbildung (2,3-Dichlornitrobenzol und Dinitrodichlorbenzole) in Verbindung mit einer für die adiabatische Reaktionsführung notwendigen hohen Reaktionsgeschwindigkeit realisieren lassen

Die Erfindung betrifft ein Verfahren zur Herstellung von 3,4-Dichlornitrobenzol durch Umsetzung von 1,2-Dichlorbenzol mit einem HNO₃/H₂SO₄/H₂O-Gemisch unter Bildung im wesentlichen der Dichlornitrobenzole und Reaktionswasser, gekennzeichnet durch die Schritte
- a): Einspeisung von 1,2-Dichlorbenzol, HNO₃, H₂SO₄ und H₂O in beliebiger Reihenfolge in einen mit Mischungsorganen ausgestatteten Reaktor, wobei
- a1): die Menge an HNO₃ 1 bis 5 Gew.-%, die Menge an H₂SO₄ 70 bis 92 Gew.-% und die Menge an H₂O den Rest zu 100 Gew.-% beträgt und 100 Gew.-% die Summe von HNO₃ + H₂SO₄ + H₂O darstellen,
- a2): das H₂O als solches, als Verdünnungs-H₂O der HNO₃, als Verdünnungs-H₂O der H₂SO₄ oder in mehreren der genannten Formen eingesetzt wird, und
- a3): das molare Verhältnis von 1,2-Dichlorbenzol zu HNO₃ 0,9 bis 1,5 beträgt,
- b): rasche und intensive Vermischung der Gesamtmenge der Reaktionsteilnehmer, wozu eine Mischleistung von 1 bis 50 Watt pro Liter des gesamten Reaktionsgemisches, bevorzugt 3 bis 30 W/l, angewandt wird,
- c): Durchführung der Umsetzung unter adiabatischen Bedingungen, wobei die Reaktionsteilnehmer mit solchen Temperaturen eingespeist werden, dass die Vermischung im Bereich von 0 bis 60°C erfolgt und die Temperatur am Ende der Reaktion 100°C nicht übersteigt,
- d): Trennung des Reaktionsgemisches nach Durchführung der Reaktion in eine organische und eine anorganische Phase und
- e): destillative Aufarbeitung der weitgehend HNO₃-freien anorganischen Phase unter Entfernung von Wasser.

Das erfindungsgemäße Verfahren kann kontinuierlich oder diskontinuierlich, bevorzugt kontinuierlich durchgeführt werden.

Zur kontinuierlichen Durchführung kann beispielsweise so vorgegangen werden: Die Gesamtmenge der Reaktionsteilnehmer wird mit Hilfe von Mischorganen rasch vermischt und als Mischung in einen Reaktor eingespeist. Die Mischungszeit bei kontinuierlicher Durchführung beträgt in der Regel weniger als 3 sek, beispielsweise 1 msek bis 2,99 sek, bevorzugt 1 msek bis 2 sek. Der Reaktor ist gegebenenfalls isoliert, verhindert weitgehend die Rückvermischung und wird adiabatisch betrieben. Zur weitgehenden Verhinderung der Rückvermischung ist der Reaktor unterteilt oder besteht aus mehreren Kammern oder Einheiten; an den Übergängen zwischen den Reaktorteilen wird das Reaktionsgemisch redispergiert. Das ausreagierte Gemisch läuft ab und wird in einem Trenngefäß getrennt; die Trennung erfolgt rasch. Die organische Phase wird wie üblich, z.B. durch Wäsche und Destillation, aufgearbeitet oder sofort einer Zweitnitrierung zugeführt. Im Allgemeinen, insbesondere bei einem Überschuss an 1,2-Dichlorbenzol, ist die abgetrennte anorganische Phase praktisch frei von Salpetersäure. Sollte dies, insbesondere bei einem Überschuss an Salpetersäure, nicht der Fall sein, kann restliche Salpetersäure in einem Nachreaktor unter Zusatz von weiterem 1,2-Dichlorbenzol im Sinne einer Reaktiv-Extraktion verbraucht werden. Die von der Salpetersäure weitgehend befreite anorganische Säurephase wird bevorzugt unter Ausnutzung der aufgenommenen Reaktionswärme und unter vermindertem Druck einer Flash-Verdampfung zugeführt. Hierbei wird Wasser aus der Säure entfernt und die Säure dabei in bevorzugter Weise auf die Eingangskonzentration für Schritt a) gebracht. Durch diese Rückführung der aufgearbeiteten anorganischen Phase (H₂SO₄, H₂O) in den Prozess ergibt sich eine Kreislauffahrweise für die H₂SO₄; es kann sinnvoll sein, einen geringen Teil dieser H₂SO₄ auszuschleusen, um etwaige Verunreinigungen auf einem niedrigen Niveau zu lassen. Für den Fall, dass die anorganische Phase noch 1,2-Dichlorbenzol, Dichlornitrobenzole und evtl. organische Nebenprodukte enthält, kann es sinnvoll sein, die anorganische Phase vor der Flash-Verdampfung zur Entfernung der Organika zu strippen. Das danach als Flash-Kondensat erhaltene Wasser ist dann von höherer Reinheit, und seine Entsorgung ist einfacher. Selbstverständlich kann auch das Flash-Kondensat durch Strippen von Organika befreit werden, wobei analog ein restliches Flash-Kondensat von höherer Reinheit zurückbleibt. Die bei der Nachreaktion der HNO₃ mit weiterem 1,2-Dichlorbenzol sowie beim Strippen oder bei der Aufkonzentrierung der weitgehend HNO₃-freien anorganischen Phase anfallenden Organika können dem Prozess an geeigneter Stelle zugesetzt werden (1,2-Dichlorbenzol, Dichlor(di)nitrobenzol) oder werden ausgeschleust und entsorgt (Verunreinigungen, Nebenprodukte).

Die Reaktionsteilnehmer können gemeinsam, jedoch auch einzeln oder als Gemische zweier oder dreier von ihnen gleichzeitig oder sukzessive dem mit Mischungsorganen ausgestatteten Reaktor zugeführt werden. Die Vermischung der Einsatzstoffe kann beispielsweise so stattfinden, dass man 1,2-Dichlorbenzol und Salpetersäure als zwei separate Ströme gleichzeitig oder sukzessive der aufkonzentrierten, gebrauchten Schwefelsäure zusetzt, wobei die Salpetersäure durch Wasser und/oder Schwefelsäure und Wasser verdünnt sein kann. Das 1,2-Dichlorbenzol kann auch mit Wasser und Schwefelsäure vorvermischt und die resultierende Emulsion mit Salpetersäure, die mit Schwefelsäure und/oder Wasser vermischt sein kann, weiter rasch und intensiv vermischt werden. Weiterhin kann auch das 1,2-Dichlorbenzol mit einer Mischsäure aus H₂SO₄, HNO₃ und H₂O intensiv vermischt werden. Noch weitere Varianten der Zuführung der Reaktionsteilnehmer, ihrer intensive Vermischung und erfindungsgemäßen Weiterbehandlung sind für den Fachmann leicht erkennbar. Dazu sind die in der Technik bekannten Mischorgane geeignet, z.B.: 1. Statische Mischer, 2. Pumpen, 3. Düsen, 4. Rührer oder Kombinationen hiervon.

Für das Gelingen der Reaktion ist es von geringerer Bedeutung, in welcher Reihenfolge und Zusammensetzung die Reaktionsteilnehmer Salpetersäure und 1,2-Dichlorbenzol sowie Schwefelsäure und Wasser miteinander vermischt werden, solange das Reaktionsgemisch nach der Gesamtvermischung die erfindungsgemäße Zusammensetzung besitzt und die Vermischung in der erfindungsgemäßen Intensität stattfindet.

Der Einspeisung und intensiven Vermischung der Reaktionsteilnehmer folgen bei kontinuierlicher Durchführung mindestens zwei Dispergierungen. Hierzu sind im Reaktor Lochbleche, Schlitzbleche, Prallbleche, Rührer oder ähnliche, dem Fachmann für diesen Zweck bekannte Einbauten bzw. Organe vorhanden.

Als kontinuierlich betriebene Reaktoren für das erfindungsgemäße Verfahren seien beispielsweise genannt: Rohrreaktoren mit Einbauten zur Redispergierung, wie Strömungsbrechern, Umlenkblechen, Statikmischern, Rührern und ähnlichen; stark gerührte Kessel in Kaskadenanordnung; Schlaufenreaktoren mit Einbauten wie oben; Kombinationen mehrerer der genannten Apparatate; weitere gleichwirkende Reaktoren, wie Kammerreaktoren mit Rührern in jeder Kammer. In bevorzugter Weise werden Rohrreaktoren mit Einbauten eingesetzt. Die Einbauten sind bevorzugt Lochbleche. Alle Einbauten stellen Unterteilungen der Gesamtapparatur dar, die gleichermaßen der Redispergierung und der weitgehenden Verhinderung der Rückvermischung dienen.

Nach dem intensiven Vermischen, nach jeder Dispergierung und nach dem Durchströmen einer gewissen Teillänge der Reaktors wird ein Koaleszieren der Dispersionströpfchen beobachtet, was durch Redispergierung rückgängig gemacht wird. Die Zahl der Dispergierungsvorgänge beträgt erfindungsgemäß 2-50, bevorzugt 3-30, besonders bevorzugt 4-20. Zur Überwindung der dabei auftretenden Druckverluste wird mit den Reaktionsteilnehmern eine Mischenergie pro Liter des gesamten Reaktionsgemisches von 1-50 Watt/Liter, bevorzugt 3-30 W/l, in das Reaktionssystem gegeben.

Die Vermischung der Reaktionsteilnehmer erfolgt im Bereich von 0 bis 60°C, bevorzugt 10 bis 50°C, besonders bevorzugt 20 bis 40°C. Es werden adiabatische Reaktionsbedingungen eingehalten. Die Endtemperatur ist abhängig von der Höhe der Mischungstemperatur, von den Mengenverhältnissen der Reaktionsteilnehmer und vom Umsatz; sie übersteigt im allgemeinen 100°C nicht, bevorzugt nicht 85°C, besonders bevorzugt nicht 70°C.

Der Gehalt an zugesetzter Salpetersäure im Reaktionsgemisch zum Zeitpunkt der Vermischung beträgt, bezogen auf die Summe von Salpetersäure, Schwefelsäure und Wasser, 1 bis 5 Gew.-%, bevorzugt 1 bis 4 Gew.-%, besonders bevorzugt 1,5 bis 3 Gew.-%. Salpetersäure kann in hochkonzentrierter oder in azeotrop siedender Form, beispielsweise als 60-98 Gew.-%ige HNO₃, bevorzugt aber in Form der etwa 60-65 Gew.-% aufweisenden und billig verfügbaren "Schwachsäure" eingesetzt werden.

Der Gehalt an Schwefelsäure im Reaktionsgemisch zum Zeitpunkt der Vermischung beträgt, bezogen auf die Summe von Salpetersäure, Schwefelsäure und Wasser, 70 bis 92 Gew.-%, bevorzugt 80 bis 90 Gew.-%, besonders bevorzugt 82 bis 88 Gew.-%.

Der Rest zu 100 Gew.-% ist H₂O. Dies kann als solches, als Verdünnungs-H₂O der H₂SO₄, als Verdünnungs-H₂O der HNO₃ oder in mehreren der genannten Formen eingesetzt werden. In bevorzugter Form liegt H₂O als Verdünnungs-H₂O sowohl der H₂SO₄ als auch der HNO₃ vor.

Das molare Verhältnis von 1,2-Dichlorbenzol zu HNO₃ beträgt allgemein 0,9 bis 1,5. Um die Bildung unerwünschter Dinitrohalogenbenzole zu minimieren, beträgt das Molverhältnis von Halogenbenzol zu Salpetersäure bevorzugt 1,0 bis 1,5, besonders bevorzugt 1,01 bis 1,3, ganz besonders bevorzugt 1,05 bis 1,2.

Es werden 1,2-Dichlorbenzol und HNO₃ in das Verfahren eingeführt und Dichlornitrobenzol und H₂O ausgeschleust, während das beschriebene H₂SO₄/H₂O-Gemisch das Reaktionsmedium darstellt. Da bei der technischen Realisierung vorteilhafterweise verdünnte Salpetersäuren eingesetzt werden, muss zusätzlich zum Reaktionswasser auch noch Verdünnungs-H₂O der HNO₃ ausgeschleust werden.

Die bei der Trennung des Reaktionsgemisches anfallende organische Phase kann auf reines 3,4-Dichlornitrobenzol aufgearbeitet werden oder der Zweitnitrierung zugeführt werden. Im ersteren Fall wird man, wie oben beschrieben, mindestens molare Mengen an 1,2-Dichlorbenzol oder einen geringen molaren Überschuss einsetzen, um sowohl die HNO₃ zu verbrauchen, als auch die Zweitnitrierung zu unterdrücken; ein etwaiger 1,2-Dichlorbenzolüberschuss wird aus der organischen Phase abdestilliert. Zuvor kann die organische Phase gewaschen werden, um wasser-, säure- oder alkalilösliche Verunreinigungen abzutrennen, wie anorganische und organische Säuren und phenolische Verunreinigungen. Die Bildung von Oxidationsprodukten (Phenolkörper) ist stark unterdrückt. Ebenso ist die Bildung von Dinitrodichlorbenzolen stark unterdrückt. Diese Dinitrodichlorbenzole stören jedoch dann nicht, wenn ohnehin eine Zweitnitrierung vorgesehen ist; daher darf in solchen Fällen auch mit einem geringen 1,2-Dichlorbenzolunterschuss gearbeitet werden.

Als Modell für einen rückvermischungsfreien technischen Reaktor und zur Darstellung der diskontinuierlichen Durchführung kann im Labor ein Batch-Ansatz in einem stark gerührten, wärmeisolierten Rührkolben, z.B. in einem sogenannten Sulfierbecher, der mit Stromstörem versehen ist, dienen. Dabei werden 1,2-Dichlorbenzol, Schwefelsäure und Wasser z.B. bei 30°C vorgelegt und mit der auf die erfindungsgemäße Einsatztemperatur erwärmten Salpetersäure, die durch Wasser und/oder Schwefelsäure verdünnt sein kann, in wenigen Sekunden versetzt. Nach der Dosierung lässt man die Reaktion adiabatisch ablaufen. Die Endtemperatur wird dabei in ca. 0,5 bis 10 Minuten erreicht. Alternativ kann auch die Gesamtmenge an HNO₃, H₂SO₄ und H₂O vorgelegt und mit 1,2-Dichlorbenzol versetzt werden; noch weitere Dosiervarianten sind für den Fachmann leicht erkennbar. Der Inhalt des Reaktionsgefäßes entspricht dabei im zeitlichen Verlauf einem Volumenteil in der axialen Bewegung durch einen Rohrreaktor mit Pfropfenströmung. Was im Batch-Ansatz zeitlich nacheinander geschieht, läuft etwa in einem Rohrreaktor örtlich hintereinander ab.

Nach Erreichen der Reaktionstemperatur wird der Rührer angehalten. Die Phasen trennen sich in maximal 10 Minuten. Die Dimensionierung eines kontinuierlichen technischen Reaktors wird bevorzugt so bemessen, dass das Reaktionsgemisch die Reaktionsendtemperatur im Reaktor erreicht.

Die nach der Reaktion auf mindestens dem Niveau der jeweiligen Reaktionsendtemperatur abgetrennte Säurephase wird in der oben beschriebenen Weise aufkonzentriert, wobei die oben beschriebene Extraktion bzw. Reaktiv-Extraktion eingeschoben werden kann. Die so geführte Kreislaufsäure enthält nach dem erfindungsgemäßen Verfahren beispielsweise weniger als 0,2 Gew.-% Salpetersäure und weniger als 0,5 Gew.-% salpetrige Säure, sowie gegebenenfalls geringe Mengen an organischen Verunreinigungen.

Das erfindungsgemäße Verfahren zeichnet sich dadurch aus , dass das unerwünschte Nebenprodukt 2,3-Dichlornitrobenzol in wesentlich geringerem Umfang entsteht, als nach den bekannten Verfahren. Werden die Reaktionspartner bei der adiabatischen Nitrierung von 1,2-Dichlorbenzol erfindungsgemäß bei 30 °C vermischt, wobei die Menge an eingesetzter HNO₃ 3,0 Gew.-% bezogen auf die Summe von eingesetztem Wasser, Salpetersäure und Schwefelsäure beträgt, so bilden sich 12,2 Gew.-% (bezogen auf die Summe der gebildeten Dichlormononitrobenzole) des unerwünschten Nebenprodukts 2,3-Dichlornitrobenzol. Erfolgt die Vermischung der Reaktionspartner jedoch gemäß Beispiel 2 aus EP 675 104 A1 bei 110 °C, wobei die Menge an eingesetzter HNO₃ unverändert 3,0 Gew.-% beträgt, so werden 16,3 Gew.-% 2,3-Dichlornitrobenzol gebildet. Gemäß dem erfindungsgemäßen Verfahren werden also fast 25 % weniger unerwünschtes Nebenprodukt gebildet. Neben der Temperatur der Reaktionspartner bei der Vermischung ist auch die Menge an eingesetzter HNO₃ für das erfindungsgemäße Verfahren von entscheidender Bedeutung. Mit zunehmendem Gehalt an HNO₃ in der eingesetzten Mischsäure erhöht sich auch der Temperaturanstieg während der adiabatischen Nitrierung, was wiederum eine verstärkte Nebenproduktbildung nach sich zieht. Im vorliegenden Beispiel 2 (1,5 Gew.-% HNO₃) werden 11,5 Gew.-% des unerwünschten 2,3-Dichlornitrobenzol gebildet, im vorliegenden Beispiel 1 (3,0 Gew.-% HNO₃) 12,2 Gew.-%. Wird eine Mischsäure mit einem Gehalt an HNO₃ über 5 Gew.-% eingesetzt, so bildet sich deutlich mehr unerwünschtes Nebenprodukt.

Anhand folgender Beispiele wird das erfindungsgemäße Verfahren weiter illustriert. Diese Beispiele stellen jedoch keine Einschränkung des Erfindungsgedankens dar.

### Beispiele

### Beispiel 1

In einer kontinuierlich betriebenen Anlage wurde ein Strom von 188,0 kg H₂SO₄ (87 Gew.-%) pro Stunde mit einem Strom von 9,56 kg HNO₃ (62 Gew.-%) pro Stunde vermischt. Die resultierende Mischsäure enthielt 3,0 Gew.-% HNO₃. Die Mischsäure wurde mit einem Strom von 15,21 kg 1,2-Dichlorbenzol pro Stunde am Eingang eines Rohrreaktors vermischt. Die Temperatur zum Zeitpunkt der Vermischung betrug 30 °C. Das Reaktionsgemisch verließ den Rohrreaktor mit einer Temperatur von 67 °C nach 3 Minuten. Anschließend wurden die organische und die anorganische Phase des Reaktionsgemischs in einem Phasenscheider voneinander getrennt.

Es wurde erhalten: 18,60 kg/h Organische Phase

Zusammensetzung (geeichte GC):

| | |
|---|---|
| 1,2-Dichlorbenzol | 7,9 Gew.-% |
| 3,4-Dichlornitrobenzol | 80,5 Gew.-% |
| 2,3-Dichlornitrobenzol | 11,5 Gew.-% |
| Dinitrodichlorbenzole | 0,03 Gew.-% |

Die Säurephase enthielt 0,84 kg/h 3,4-Dichlornitrobenzol und 0,07 kg/h 2,3-Dichlornitrobenzol.

Damit betrug der Gesamt-Anteil von 2,3-Dichlornitrobenzol bezogen auf die Summe der gebildeten Dichlormononitrobenzole 12,2 Gew.-%.

### Beispiel 2

In einer kontinuierlich betriebenen Anlage wurde ein Strom von 188,0 kg H₂SO₄ (88 Gew.-%) pro Stunde mit einem Strom von 4,66 kg HNO₃ (62 Gew.-%) pro Stunde vermischt. Die resultierende Mischsäure enthielt 1,5 Gew.-% HNO₃. Die Mischsäure wurde mit einem Strom von 6,74 kg 1,2-Dichlorbenzol pro Stunde am Eingang eines Rohrreaktors vermischt. Die Temperatur zum Zeitpunkt der Vermischung betrug 30 °C. Das Reaktionsgemisch verließ den Rohrreaktor mit einer Temperatur von 48 °C nach 3 Minuten.

Nach der Phasentrennung wurde erhalten:
7,20 kg/h Organische Phase

Zusammensetzung (geeichte GC):

| | |
|---|---|
| 1,2-Dichlorbenzol | 3,6 Gew.-% |
| 3,4-Dichlornitrobenzol | 84,4 Gew.-% |
| 2,3-Dichlornitrobenzol | 11,9 Gew.-% |
| Dinitrodichlorbenzole | 0,08 Gew.-% |

Die Säurephase enthielt 1,48 kg/h 3,4-Dichlornitrobenzol und 0,13 kg/h 2,3-Dichlomitrobenzol.

Damit betrug der Gesamt-Anteil von 2,3-Dichlornitrobenzol bezogen auf die Summe der gebildeten Dichlormononitrobenzole 11,5 Gew.-%.

### Beispiel 3

In einer kontinuierlich betriebenen Anlage wurde ein Strom von 141,0 kg H₂SO₄ (86,6 Gew.-%) pro Stunde mit einem Strom von 5,31 kg HNO₃ (62 Gew.-%) pro Stunde vermischt. Die resultierende Mischsäure enthielt 2,25 Gew.-% HNO₃. Die Mischsäure wurde mit einem Strom von 8,45 kg 1,2-Dichlorbenzol pro Stunde am Eingang eines Rohrreaktors vermischt. Die Temperatur zum Zeitpunkt der Vermischung betrug 30 °C. Das Reaktionsgemisch verließ den Rohrreaktor mit einer Temperatur von 57 °C nach 3 Minuten.

Nach der Phasentrennung wurde erhalten:
10,04 kg/h Organische Phase

Zusammensetzung (geeichte GC):

| | |
|---|---|
| 1,2-Dichlorbenzol | 8,4 Gew.-% |
| 3,4-Dichlornitrobenzol | 80,4 Gew.-% |
| 2,3-Dichlornitrobenzol | 11,2 Gew.-% |
| Dinitrodichlorbenzole | 0,04 Gew.-% |

Die Säurephase enthielt 0,75 kg/h 3,4-Dichlornitrobenzol und 0,06 kg/h 2,3-Dichlomitrobenzol.

Damit betrug der Gesamt-Anteil von 2,3-Dichlornitrobenzol bezogen auf die Summe der gebildeten Dichlormononitrobenzole 11,8 Gew.-%.

### Beispiel 4

In einem wärmeisolierten Sulfierbecher (⌀ 100 mm), versehen mit Stromstörern und zwei auf einer Welle sitzenden Turbinenrührern (⌀ 39,9 mm) wurden 667,8 g H₂SO₄ (86,6 %ig) und 32,3 g (0,333 mol) HNO₃ (65 %ig) bei 30 °C unter Rühren vorgelegt (eingebrachte spezifische Rührleistung 22 W/l) und in 3 Sekunden mit 49,0 g (0,333 mol) 1,2-Dichlorbenzol mit einer Temperatur von 30 °C versetzt, und man ließ ohne Kühlung reagieren. Nach 170 Sekunden hatte das Reaktionsgemisch die Endtemperatur von 67 °C erreicht und der Rührer wurde angehalten. Nach Phasentrennung wurden 58,3 g organische Phase erhalten.

| | |
|---|---|
| 1,2-Dichlorbenzol | 1,2 Gew.-% |
| 3,4- Dichlornitrobenzol | 86,4 Gew.-% |
| 2,3-Dichlornitrobenzol | 12,3 Gew.-% |
| Dinitrodichlorbenzole | 0,03 Gew.-% |

Aus der Säurephase ließen sich 4,70 g 3,4-Dichlornitrobenzol und 0,49 g 2,3-Dichlomitrobenzol isolieren.

Damit betrug der Gesamt-Anteil von 2,3-Dichlornitrobenzol bezogen auf die Summe der gebildeten Dichlormononitrobenzole 12,2 Gew.-%.

## Patentansprüche

1. Verfahren zur Herstellung von 3,4-Dichlornitrobenzol durch Umsetzung von 1,2-Dichlorbenzol mit einem HNO₃/H₂SO₄/H₂O-Gemisch unter Bildung im wesentlichen der Dichlornitrobenzole und Reaktionswasser, **gekennzeichnet durch** die Schritte
a) Einspeisung von 1,2-Dichlorbenzol, HNO₃, H₂SO₄ und H₂O in beliebiger Reihenfolge in einen mit Mischungsorganen ausgestatteten Reaktor, wobei
a1) die Menge an HNO₃ 1 bis 5 Gew.-%, die Menge an H₂SO₄ 70 bis 92 Gew.-% und die Menge an H₂O den Rest zu 100 Gew.-% beträgt und 100 Gew.-% die Summe von HNO₃ + H₂SO₄ + H₂O darstellen,
a2) das H₂O als solches, als Verdünnungs-H₂O der HNO₃, als Verdünnungs-H₂O der H₂SO₄ oder in mehreren der genannten Formen eingesetzt wird, und
a3) das molare Verhältnis von 1,2-Dichlorbenzol zu HNO₃ 0,9 bis 1,5 beträgt,
b) rasche und intensive Vermischung der Gesamtmenge der Reaktionsteilnehmer, wozu eine Mischleistung von 1 bis 50 Watt pro Liter des gesamten Reaktionsgemisches, bevorzugt 3 bis 30 W/l, angewandt wird,
c) Durchführung der Umsetzung unter adiabatischen Bedingungen wobei die Reaktionsteilnehmer mit solchen Temperaturen eingespeist werden, dass die Vermischung im Bereich von 0 bis 60°C erfolgt und die Temperatur am Ende der Reaktion 100°C nicht übersteigt,
d) Trennung des Reaktionsgemisches nach Durchführung der Reaktion in eine organische und eine anorganische Phase und
e) destillative Aufarbeitung der weitgehend HNO₃-freien anorganischen Phase unter Entfernung von Wasser.

2. Verfahren gemäß Anspruch 1, **dadurch gekennzeichnet, dass** die Umsetzung kontinuierlich durchgeführt wird.

3. Verfahren nach mindestens einem der Ansprüche 1 und 2, **dadurch gekennzeichnet, dass** die Reaktionsteilnehmer vor Eintritt in einen die Rückvermischung weitgehend verhindernden Reaktor mit Hilfe von Mischorganen innerhalb einer Zeit von weniger als 3 sek. intensiv gemischt werden.

4. Verfahren nach Anspruch 1, **dadurch gekennzeichnet, dass** im Schritt e) die infolge der adiabatischen Reaktionsführung von der anorganischen Phase aufgenommene Reaktionswärme zur destillativen Ausschleusung von Wasser unter einem Druck von 40 bis 150 mbar, bevorzugt 40 bis 120 mbar, besonders bevorzugt 50-100 mbar, ausgenutzt wird und bevorzugt dabei die Konzentration der ablaufenden H₂SO₄ so eingestellt wird, dass diese ablaufende H₂SO₄ zum Einsatz in Schritt a) geeignet ist.

5. Verfahren gemäß Anspruch 1, **dadurch gekennzeichnet, dass** der Gehalt an zugesetzter Salpetersäure im Reaktionsgemisch zum Zeitpunkt der Vermischung, bezogen auf die Summe von Salpetersäure, Schwefelsäure und Wasser, 1 bis 4 Gew.-% beträgt.

6. Verfahren gemäß Anspruch 5, **dadurch gekennzeichnet, dass** der Gehalt an zugesetzter Salpetersäure im Reaktionsgemisch zum Zeitpunkt der Vermischung, bezogen auf die Summe von Salpetersäure, Schwefelsäure und Wasser, 1,5 bis 3 Gew.-% beträgt.

7. Verfahren gemäß Anspruch 1, **dadurch gekennzeichnet, dass** der Gehalt an Schwefelsäure im Reaktionsgemisch zum Zeitpunkt der Vermischung, bezogen auf die Summe von Salpetersäure, Schwefelsäure und Wasser, 80 bis 90 Gew.-% beträgt.

8. Verfahren gemäß Anspruch 7, **dadurch gekennzeichnet, dass** der Gehalt an Schwefelsäure im Reaktionsgemisch zum Zeitpunkt der Vermischung, bezogen auf die Summe von Salpetersäure, Schwefelsäure und Wasser, 82 bis 88 Gew.-% beträgt.

9. Verfahren gemäß Anspruch 1, **dadurch gekennzeichnet, dass** das Molverhältnis von 1,2-Dichlorbenzol zu Salpetersäure 1,0 bis 1,5 beträgt.

10. Verfahren gemäß Anspruch 1, **dadurch gekennzeichnet, dass** die Vermischung der Reaktionsteilnehmer bei einer Temperatur von 10 bis 50 °C durchgeführt wird.

## Claims

1. A process for preparing 3,4-dichloronitrobenzene by reacting 1,2-dichlorobenzene with a HNO₃/H₂SO₄/H₂O mixture with the formation of substantially dichloronitrobenzenes and water of reaction, **characterised by** the steps
a) feeding 1,2-dichlorobenzene, HNO₃, H₂SO₄ and H₂O in any order into a reactor fitted with a mixing device, wherein
a1) the amount of HNO₃ is 1 to 5 wt.%, the amount of H₂SO₄ is 70 to 92 wt.% and the amount of H₂O makes up the remainder to 100 wt.%, and 100 wt.% is the sum of HNO₃ + H₂SO₄ + H₂O,
a2) the H₂O is used as such, as dilution H₂O in the HNO₃, as dilution H₂O in the H₂SO₄ or in several of the forms mentioned, and
a3) the molar ratio of 1,2-dichlorobenzene to HNO₃ is 0.9 to 1.5,
b) rapid and intensive mixing of the entire amount of the reaction partners, with a mixing power of 1 to 50 Watts per litre of total reaction mixture, preferably 3 to 30 W/l, being applied,
c) performing the reaction under adiabatic conditions, wherein the reaction partners are supplied at temperatures such that mixing takes place in the range 0 to 60°C and the temperature at the end of the reaction does not exceed 100°C,
d) separating the reaction mixture, after performing the reaction, into an organic and an inorganic phase and
e) working up the largely HNO₃-free inorganic phase by distillation with the removal of water.

2. A process according to Claim 1, **characterised in that** the reaction is performed continuously.

3. A process according to at least one of Claims 1 and 2, **characterised in that** the reaction partners are intensively mixed before entrance into a reactor which largely prevents back-mixing with the aid of mixing devices for a period of less than 3 sec.

4. A process according to Claim 1, **characterised in that** in step e), the heat of reaction absorbed by the inorganic phase due to adiabatic management of the reaction is used to distil out the water at a pressure of 40 to 150 mbar, preferably 40 to 120 mbar, particularly preferably 50 - 100 mbar and that the concentration of H₂SO₄ then being discharged is preferably adjusted so that this H₂SO₄ being discharged is suitable for use in step a).

5. A process according to Claim 1, **characterised in that** the concentration of added nitric acid in the reaction mixture at the time of mixing is 1 to 4 wt.%, with respect to the sum of nitric acid, sulfuric acid and water.

6. A process according to Claim 5, **characterised in that** the concentration of added nitric acid added in the reaction mixture at the time of mixing is 1.5 to 3 wt.%, with respect to the sum of nitric acid, sulfuric acid and water.

7. A process according to Claim 1, **characterised in that** the concentration of sulfuric acid in the reaction mixture at the time of mixing is 80 to 90 wt.%, with respect to the sum of nitric acid, sulfuric acid and water.

8. A process according to Claim 7, **characterised in that** the concentration of sulfuric acid in the reaction mixture at the time of mixing is 82 to 88 wt.%, with respect to the sum of nitric acid, sulfuric acid and water.

9. A process according to Claim 1, **characterised in that** the molar ratio of 1,2-dichlorobenzene to nitric acid is 1.0 to 1.5.

10. A process according to Claim 1, **characterised in that** mixing the reaction partners is performed at a temperature of 10 to 50°C.

## Revendications

1. Procédé pour la préparation du 3,4-dichloronitrobenzène par réaction du 1,2-dichlorobenzène avec un mélange HNO₃/H₂SO₄/H₂O avec formation essentiellement de dichloronitrobenzènes et d'eau de réaction, **caractérisé par** les stades opératoires suivants :
a) introduction du 1,2-dichlorobenzène, de HNO₃, de H₂SO₄ et de H₂O dans un ordre quelconque dans un réacteur équipé d'organes de mélange, dans les conditions suivantes :
a1) la quantité de HNO₃ représente 1 à 5 % en poids, la quantité de H₂SO₄ 70 à 92 % en poids et la quantité de H₂O le solde à 100 % en poids, 100 % en poids représentant la somme de HNO₃ + H₂SO₄ + H₂O,
a2) l'eau est mise en oeuvre telle quelle, sous la forme d'eau de dilution de HNO₃, sous la forme d'eau de dilution de H₂SO₄ ou sous plusieurs de ces formes, et
a3) le rapport molaire 1,2-dichlorobenzène/HNO₃ va de 0,9 à 1,5,
b) on soumet tous les réactifs à mélange rapide et intensif en apportant une puissance de mélange de 1 à 50 W/I du mélange de réaction total, de préférence de 3 à 30 W/I,
c) on exécute la réaction dans des conditions adiabatiques, les composants étant introduits à des températures telles que le mélange se produit dans l'intervalle de 0 à 60°C et que la température en fin de réaction ne dépasse pas 100°C,
d) après la réaction, on sépare le mélange de réaction en une phase organique et une phase inorganique et
e) on soumet la phase inorganique pratiquement exempte de HNO₃ à distillation avec élimination de l'eau.

2. Procédé selon la revendication 1, **caractérisé en ce que** la réaction est réalisée en continu.

3. Procédé selon au moins une des revendications 1 et 2, **caractérisé en ce que** les composants, avant entrée dans un réacteur évitant pratiquement les mélanges en retour, sont soumis à mélange intensif à l'aide d'organes de mélange en une durée inférieure à 3 s.

4. Procédé selon la revendication 1 **caractérisé en ce que**, au stade e), la chaleur de réaction absorbée par la phase inorganique en raison de la conduite de réaction adiabatique est exploitée pour éliminer l'eau par distillation sous une pression de 40 à 150 mbar, de préférence de 40 à 120 mbar et plus spécialement de 50 à 100 mbar, la concentration du H₂SO₄ sortant de cette distillation étant réglée de préférence en sorte que ce H₂SO₄ puisse être mis en oeuvre au stade a).

5. Procédé selon la revendication 1, **caractérisé en ce que** la teneur en acide nitrique ajouté dans le mélange de réaction au moment du mélange est de 1 à 4 % du poids total de l'acide nitrique, de l'acide sulfurique et de l'eau.

6. Procédé selon la revendication 5, **caractérisé en ce que** la teneur en acide nitrique ajouté dans le mélange de réaction au moment du mélange représente de 1,5 à 3 % du poids total de l'acide nitrique, de l'acide sulfurique et de l'eau.

7. Procédé selon la revendication 1, **caractérisé en ce que** la teneur en acide sulfurique dans le mélange de réaction au moment du mélange représente de 80 à 90 % du poids total de l'acide nitrique, de l'acide sulfurique et de l'eau.

8. Procédé selon la revendication 7, **caractérisé en ce que** la teneur en acide sulfurique dans le mélange de réaction au moment du mélange représente de 82 à 88 % du poids total de l'aide nitrique, de l'acide sulfurique et de l'eau.

9. Procédé selon la revendication 1, **caractérisé en ce que** le rapport molaire 1,2-dichlorobenzène/acide nitrique va de 1,0 à 1,5.

10. Procédé selon la revendication 1, **caractérisé en ce que** le mélange des réactifs est réalisé à une température de 10 à 50°C.
